(19) Europäisches Patentamt
European Patent Office
Office européen des brevets

(11) Veröffentlichungsnummer: **0 189 059 B1**

(12) **EUROPÄISCHE PATENTSCHRIFT**

(45) Veröffentlichungstag der Patentschrift: **11.03.92**

(51) Int. Cl.5: **G03C 1/815**, //C08F20/36, C07C253/00

(21) Anmeldenummer: **86100207.9**

(22) Anmeldetag: **09.01.86**

Die Akte enthält technische Angaben, die nach dem Eingang der Anmeldung eingereicht wurden und die nicht in dieser Patentschrift enthalten sind.

(54) **Lichtempfindliches, stabilisiertes fotografisches Aufzeichnungsmaterial.**

(30) Priorität: **19.01.85 DE 3501722**

(43) Veröffentlichungstag der Anmeldung:
**30.07.86 Patentblatt 86/31**

(45) Bekanntmachung des Hinweises auf die Patenterteilung:
**11.03.92 Patentblatt 92/11**

(84) Benannte Vertragsstaaten:
**BE DE FR GB**

(56) Entgegenhaltungen:
**GB-A- 2 118 315**

(73) Patentinhaber: **Agfa-Gevaert AG**
**W-5090 Leverkusen 1(DE)**

(72) Erfinder: **Helling, Günter, Dr.**
**In der Hildscheid 16**
**W-5068 Odenthal(DE)**
Erfinder: **Sobel, Johannes, Dr.**
**Willi-Baumeister-Strasse 9**
**W-5090 Leverkusen 1(DE)**
Erfinder: **Langen, Hans, Dr.**
**Weidengarten 16**
**W-5300 Bonn(DE)**

**EP 0 189 059 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein fotografisches Aufzeichnungsmaterial mit wenigstens einer lichtempfindlichen Silberhalogenidemulsionsschicht, gegebenenfalls weiteren Schichten sowie wenigstens einem in polymerer Form vorliegenden Stabilisator.

Es ist bekannt, stabilisierende Zusätze in Silberhalogenidmaterialien zur Verbesserung der Stabilität einzubringen. Durch UV-Absorber wird z.B. der unerwünschte Einfluß von UV-Licht bei der Belichtung oder durch elektrostatische Aufladungen vermieden. Es ist auch möglich, Stabilisatoren zur Verbesserung der Lichtbeständigkeit gegen sichtbares Licht einzusetzen. Durch sogenannte Stabilisatoren und Antischleiermittel kann der in Silberhalogenidmaterialien auftretende Schleier unterdrückt oder verbessert werden. UV-Absorber, Lichtstabilisatoren sowie Stabilisatoren und Antischleiermittel zur Beeinflussung des Schleiers liegen in fotografischen Aufzeichnungsmaterialien in uniformer Verteilung vor.

Derartige stabilisierende Zusätze können z.B. in gelöster Form oder in einem Ölformer, beispielsweise Trikresylphosphat, in das Aufzeichnungsmaterial eingebracht werden. Hierbei treten aber zum Teil erhebliche Nachteile auf. Beispielsweise sind Aminoallylidenmalodinitrile als UV-Absorber für fotografische Aufzeichnungsmaterialien bekannt. Sie neigen aber leicht zur Aggregatbildung, was eine unerwünscht breite Absorptionsbande mit niedriger Absorption zur Folge hat. Die bis in den blauempfindlichen Spektralbereich reichenden Flanken der Absorptionsbande verursachen daher eine gelbe Anfärbung des fertigen Bildes und eine verringerte Blauempfindlichkeit des Aufzeichnungsmaterials. Man hat versucht, dieses Problem dadurch zu lösen, daß der UV-Absorber in hochverteilter Form in einen sogenannten beladbaren Latex eingebracht wird, s. DE-A 2 541 230 und 2 541 274. Durch dieses Verfahren wird die Absorption des UV-Absorbers stärker und kürzerwellig. Dieses Verfahren ist jedoch unbefriedigend.

Es ist weiterhin bekannt, fotografisch nützliche Verbindungen in polymerer Form in Aufzeichnungsmaterialien einzubringen. Die Verwendung polymerer UV-Absorber ist bekannt aus DE-A 3 313 574, 3 327 464, US 4 307 184, DE-A 3 401 455. Die Verwendung polymerisierter Antischleiermittel ist bekannt aus DE-A 3 341 352. Die Verwendung polymerer Kuppler ist z.B. bekannt aus DE-A 3 401 455, DE-A 3 331 743, DE-A 3 148 125 und US 4 435 503.

Auch diese Form der Einbringung befriedigt noch nicht alle Ansprüche. Die aus der US 4 307 184 bekannten Polymeren sind z.B. wasserlösliche Produkte, die die Filmfestigkeit wegen hoher Quellung verschlechtern oder wasserunlösliche feste Produkte, die aufwendig emulgiert werden müssen. Im allgemeinen ist eine außerordentliche hohe Emulgatormenge erforderlich, so daß eine sehr hohe Schichtbelastung mit Emulgatoren auftritt. Hohe Emulgatormengen haben neben der hohen Schichtbelastung weitere ungünstige Eigenschaften, z.B. unbefriedigende Schärfe, Schäumen bei der Verarbeitung, Verschlechterung der Bruchfestigkeit und Ausschwitzen bei der Lagerung.

Polymere Stabilisatoren der wiederkehrenden Einheit

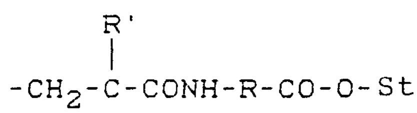

mit R = $-(CH_2)_5-$ oder Phenylen
sind aus GB-A 2 118 315 bekannt. Diese Verbindungen bedingen Nachteile in den mechanischen Eigenschaften der Materialien.

Der Erfindung liegt die Aufgabe zugrunde, ein verbessertes fotografisches Aufzeichnungsmaterial mit stabilisierenden Zusätzen aufzufinden, welches diese Nachteile nicht aufweist.

Es wurde nun ein Stabilisator und ein fotografisches Aufzeichnungsmaterial mit wenigstens einer lichtempfindlichen Silberhalogenidemulsionsschicht, gegebenenfalls weiteren Schichten sowie wenigstens einem in polymerer Form vorliegenden Stabilisator gefunden. Erfindungsgemäß weist der Stabilisator wiederkehrende Strukturelemente folgender Formel (I) auf

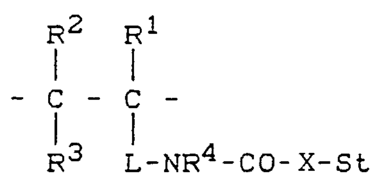

(I)

worin bedeuten

$R^1$, $R^3$, $R^4$ gleich oder verschieden, H oder Alkyl mit 1-4 C-Atomen

$R^2$ H oder Alkyl mit 1-4 C-Atomen oder $COOR^1$,

L eine chemische Bindung oder ein zweiwertiges Bindeglied,

X O oder $NR^4$,

St einen Rest mit stabilisierender Wirkung.

Unter Stabilisatoren werden für die Zwecke der vorliegenden Erfindung Verbindungen verstanden, die die Eigenschaften des Aufzeichnungsmaterials stabilisieren, insbesondere Stabilisatoren gegen die schädliche Einwirkung von sichtbarem Licht, UV-Licht, Wärme und Feuchtigkeit; Formalinfänger, Stabilisatoren und Antischleiermittel zur Unterdrückung des Schleiers und optische Aufheller.

In einer bevorzugten Ausführungsform sind $R^1$, $R^2$ und $R^4$ und gegebenenfalls $R^3$ Wasserstoff.

In einer bevorzugten Ausführungsform bedeuten:

L eine chemische Bindung oder die Gruppe $-L^1-L^2-$

$L^1$ $-CONR^1-$, $-NR^1CO-$, -CO-, -COO-, $-SO_2-$, -O- oder eine chemische Bindung

$L^2$ gegebenenfalls substituiertes Alkylen mit 1 bis 8 C-Atomen, gegebenenfalls substituiertes Arylen oder eine chemische Bindung.

In einer besonders bevorzugten Ausführungsform handelt es sich bei dem Rest St um eine UV-Licht absorbierende Einheit.

Geeignete UV-Licht absorbierende Strukturen sind insbesondere: Aminoallylidenmalonitrilderivate, Hydroxyphenylbenzotriazolderivate, Benzophenonderivate, Benzaldehydderivate, substituierte Acrylsäurederivate, Thiazolidonderivate, $\alpha$- und $\gamma$-Benzopyronderivate, Benzthiadiazolderivate, Arylidenfluorene und heterocyclische Azine.

Geeignete optische Aufheller sind z.B. Stilbenverbindungen Cumarinderivate, 1,3-Diphenylpyrazolin-Derivate, Naphthalimid-Derivate, Benzoxazol-Derivate.

Besonders geeignete Verbindungen gegen die schädliche Einwirkung von sichtbaren Licht, Wärme und Feuchte sind insbesondere alkylierte Monophenole, alkylierte Hydrochinone und Hydrochinonether, hydroxylierte Thiodiphenylether, Alkyliden-Bisphenole, Benzylverbindungen, Acylaminophenole, Ester der $\beta$-(3,5-Di-tert.-butyl-4-hydroxyphenyl)-propionsäure mit ein- oder mehrwertigen Alkoholen, Ester der $\beta$-(5-tert.-Butyl-4-hydroxy-3-methylphenyl)-propionsäure mit ein- oder mehrwertigen Alkoholen, Amide der $\beta$-(3,5-Di-ter.-butyl-4-hydroxyphenyl)-propionsäure, Ester von gegebenenfalls substituierten Benzoesäuren, Nickelverbindungen, sterisch gehinderte Amine, Oxalsäurediamide, Phosphite und Phosphonite und Hydroxychromane, Hydroxycumarane und ihre Bisspiroverbindungen.

Geeignete Verbindungen zur Verbesserung der Formalinbeständigkeit sind insbesondere in der US-Patentschrift Nr. 4.464 463 zusammenfassend angegeben.

Besonders geeignete stabilisatoren und Antischleiermittel zur Unterdrückung des Silberhalogenidschleiers sind insbesondere Azaindene, Benzotriazole, Tetrazole, Acetylenderivate und Mercaptoazole.

Besonders bevorzugte Reste St sind:

1) 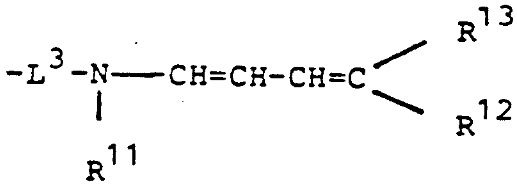

2) 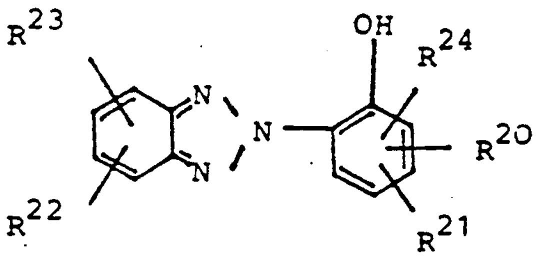

3) 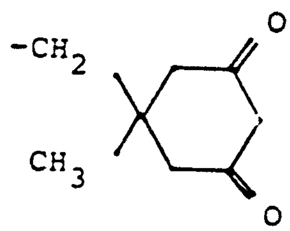

4) 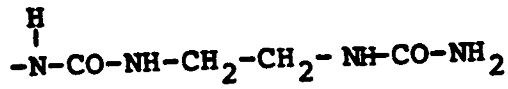

5) 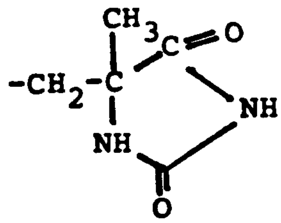

6) 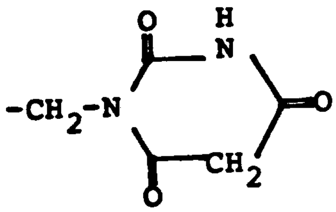

7) 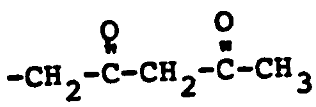

8) 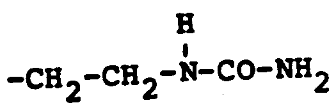

9) 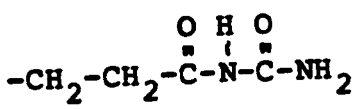

10) 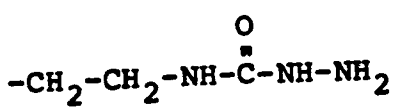

11) 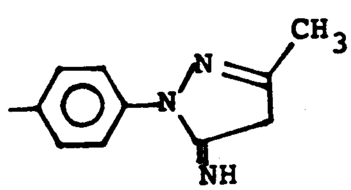

12) 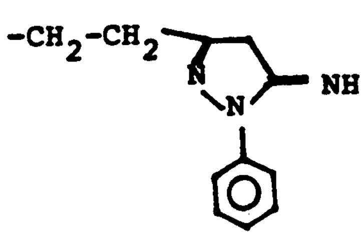

worin bedeuten

- $L^3$ eine verbindende Gruppe, ausgewählt aus einer Alkylengruppe mit 1 bis 20 Kohlenstoffatomen (beispielsweise eine Methylengruppe, eine Ethylengruppe, eine Trimethylengruppe, eine 2-Hydroxytrimethylengruppe, eine Pentamethylengruppe, eine Hexamethylengruppe, eine Ethylethylengruppe, eine Propylengruppe,
- $R^{11}$ eine Alkylgruppe mit 1 bis 6 Kohlenstoffatomen (z.B. Methyl, Ethyl, n-Propyl, i-Propyl, Cyclohexyl),
- $R^{12}$ eine Cyanogruppe, $-COOR^{14}$, $-CONHR^{14}$, $-COR^{14}$ oder $-SO_2R^{14}$,
- $R^{13}$ eine Cyanogruppe, $-COOR^{15}$, $-CONHR^{15}$, $-COR^{15}$ oder $-SO_2R^{15}$,
- $R^{14}$, $R^{15}$ gleich oder verschieden, z.B. eine gegebenenfalls substituierte Alkylgruppe mit 1 bis 20 C-Atomen (beispielsweise eine Methylgruppe, eine Ethylgruppe, eine n-Butylgruppe, eine n-Hexylgruppe, eine Cyclohexylgruppe, eine n-Decylgruppe, eine n-Dodecylgruppe, eine n-Octadecylgruppe, eine Eicosylgruppe, eine Methoxyethylgruppe, eine Ethoxypropylgruppe, eine 2-Ethylhexylgruppe, eine Hydroxyethylgruppe, eine Chlorpropylgruppe, eine N,N-Diethylaminopropylgruppe, eine Cyanoethylgruppe, eine Phenethylgruppe, eine Benzylgruppe, eine p-tert.-Butylphenethylgruppe, eine p-tert.-Octylphenoxyethylgruppe, eine 3-(2,4-di-tert.-Amylphenoxy)-propylgruppe, eine Ethoxycarbonylmethylgruppe, eine 2-(2-Hydroxyethoxy)-ethylgruppe, eine 2-Furylethylgruppe), oder eine Arylgruppe mit 6 bis 20 Kohlenstoffatomen (beispielsweise eine Tolylgruppe, eine Phenylgruppe, eine Anisylgruppe, eine Mesitylgruppe, eine Chlorphenylgruppe, eine 2,4-di-tert.-Amylphenylgruppe, eine Naphthylgruppe, darstellen, darüber hinaus können $R^{14}$ und $R^{15}$ unter Bildung eines 5- oder 6-gliedrigen Ringes kombiniert sein, beispielsweise unter Bildung von einem 1,3-Dioxocyclohexanring (beispielsweise einen Dimedonring, einen 1,3-Dioxo-5,5-diethylcyclohexanring), einen 1,3-Diaza-2,4,6-trioxocyclohexanring (beispielsweise einen Barbitursäurering, einen 1,3-Dimethylbarbitursäurering, einen 1-Phenylbarbitursäurering, einen 1-Methyl-3-octylbarbitursäurering, einen 1-Ethyl-3-octyloxycarbonylethylbarbitursäurering,), einen 1,2-Diaza-3,5-dioxocyclopentanring (beispielsweise einen 1,2-Diaza-1,2-dimethyl-3,5-dioxocyclopentanring, einen 1,2-Diaza-1,2-diphenyl-3,5-dioxocyclopentanring) oder einen 2,4-Diaza-1-alkoxy-3,5-dioxocyclohexenring (beispielsweise einen 2,4-Diaza-1-ethoxy-4-ethyl-3,5-dioxocyclohexenring, einen 2,4-Diaza-1-ethoxy-4-[3-(2,4-di-tert.-amylphenoxy)-propyl]-3,5-dioxocyclohexenring)
- $R^{20}$ - $R^{24}$, gleich oder verschieden, jeweils H, Halogen, $NO_2$, Alkyl- oder Alkoxygruppe mit 1 bis 18 C-Atomen (gegebenenfalls substituiert), Aryl oder Aryloxygruppe (gegebenenfalls substituiert), Amino (gegebenenfalls substituiert).

Einer der Reste $R^{20}$-$R^{24}$ bedeutet eine chemische Bindung oder ein zweiwertiges chemisches Bindeglied wie Alkylen mit 1 bis 8 C-Atomen, Arylen (gegebenenfalls substituiert).

Besonders bevorzugte Verbindungen der Formel (I) weisen folgende Struktur auf

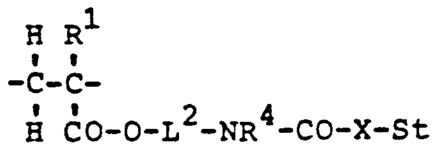

(II)

worin bedeuten

- $R^1$ H, $CH_3$,
- $R^4$ H, $CH_3$
- $L^2$ Alkylen, insbesondere Ethylen, Butylen, Propylen und worin
- St die oben angegebene Bedeutung hat und insbesondere ein UV-Absorber ist.
- X O oder $NR^1$

Ein besonders bevorzugter UV-Absorbertyp hat folgende Struktur

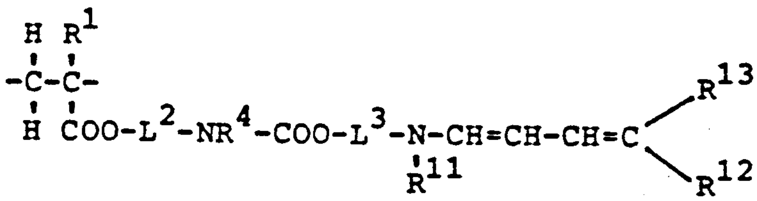

(III)

worin die Substituenten folgende Bedeutung haben

$R^1$ H, $CH_3$,

$R^4$ H, $CH_3$,

$L^2$ und $L^3$ gleich oder verschieden, Ethylen, Propylen oder Butylen,

$R^{11}$ eine gegebenenfalls substituierte Alkylgruppe, insbesondere mit 1-6 C-Atomen,

$R^{12}$ eine Cyanogruppe oder $-SO_2R^{14}$,

$R^{13}$ CN oder $COOR^{15}$,

$R^{14}/R^{15}$ gleich oder verschieden eine gegebenenfalls substituierte Alkylgruppe mit 1 bis 20 C-Atomen, vorzugsweise mit 1 bis 6 C-Atomen oder eine gegebenenfalls substituierte Arylgruppe mit 6 bis 20 C-Atomen.

In einer besonders bevorzugten Ausführungsform bedeutet X in den Formeln I-II Sauerstoff.

Verbindungen mit wiederkehrenden Struktureinheiten der Formel (I) lassen sich herstellen durch übliche Polymerisation der entsprechenden Ausgangsmonomeren folgender Formel Ia

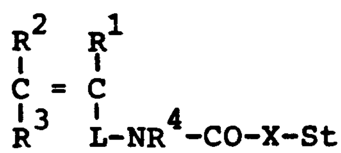

(Ia)

worin die Substituenten die zu Formel I angegebene Bedeutung haben.

Derartige Monomere lassen sich herstellen durch Umsetzung von hydroxyl- oder aminogruppenhaltigen Stabilisatoren mit ethylenisch ungesättigten Isocyanaten.

Herstellung des Monomeren M 1

36 g 3-(N-Methyl-N-2-hydroxyethyl)-amino)allylidenmalonitril werden mit 31 g Isocyanato-ethyl-methacrylat in 250 ml Essigester unter Zusatz von 1 g 1,4-Diazabicyclo[2,2,2]octan und 0,5 g Jonol (2,6-Di-tert.-butyl-4-methylphenol) 3 Stunden bei 25-30 °C gerührt und danach über Nacht stehen gelassen. Die Lösung wird im Vakuum eingeengt, der gelbliche Rückstand in 70 ml Methanol unter Zusatz von 0,5 g Jonol bei 30 °C gelöst und danach auf 0 °C abgekühlt. Der Niederschlag wird abfiltriert und im Vakuum bei Zimmertemperatur getrocknet. Es werden 34 g eines gelblichen Produktes vom Schmelzpunkt 73-74 °C erhalten.

Besonders bevorzugte spezielle Monomere sind im folgenden angegeben:

M 1 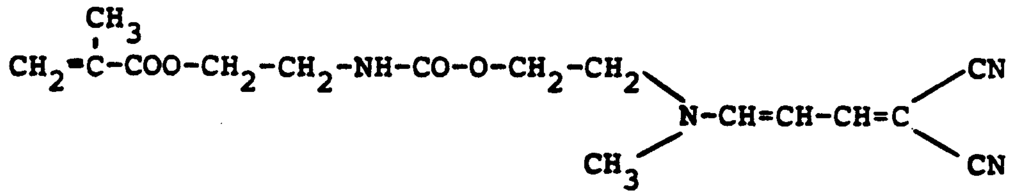

M 2 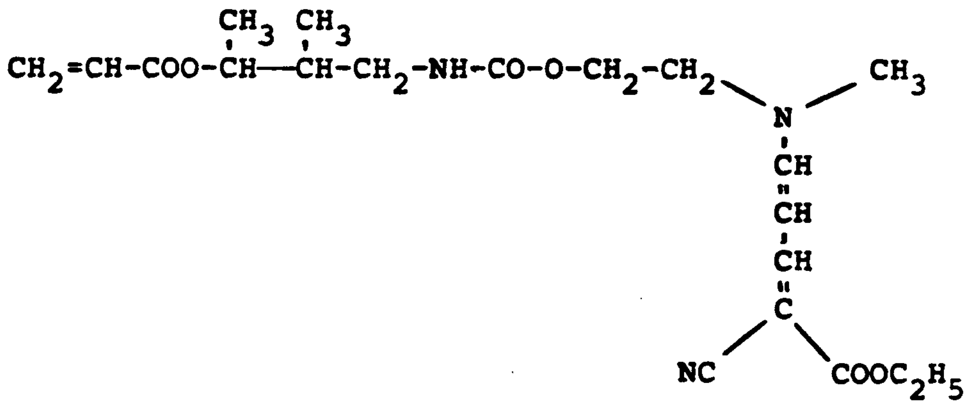

M 3 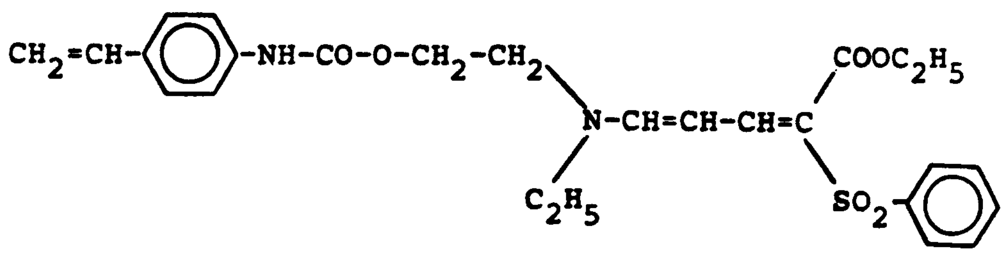

M 4 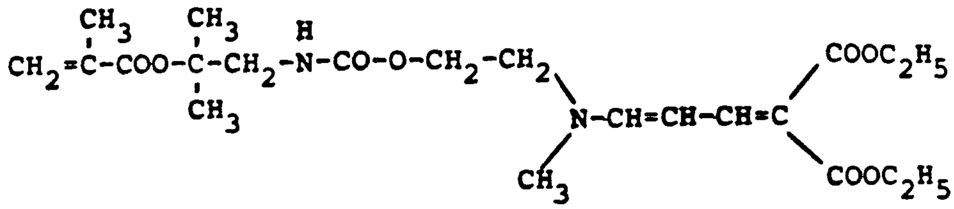

M 5 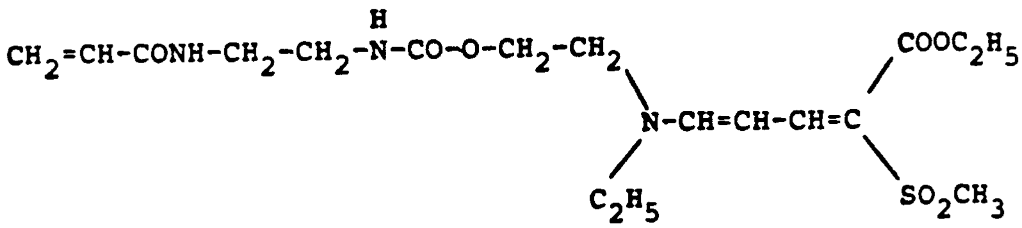

M 6 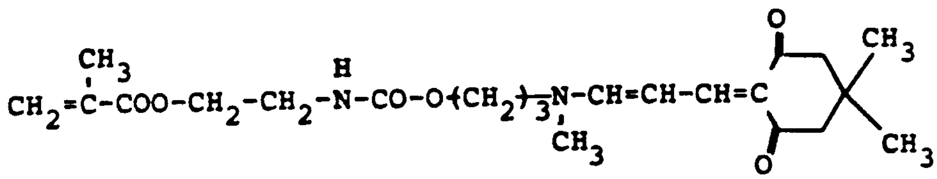

M 7 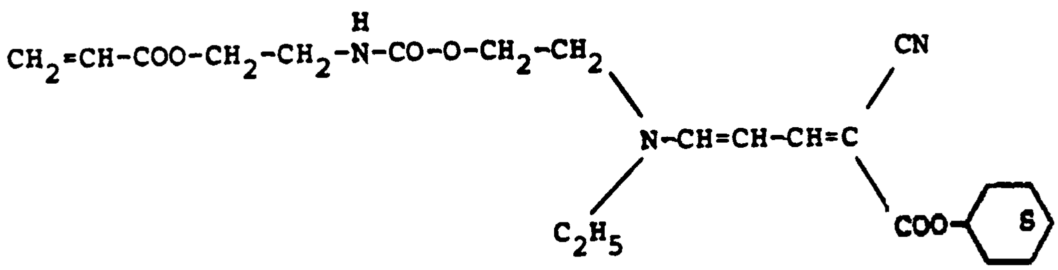

M 8 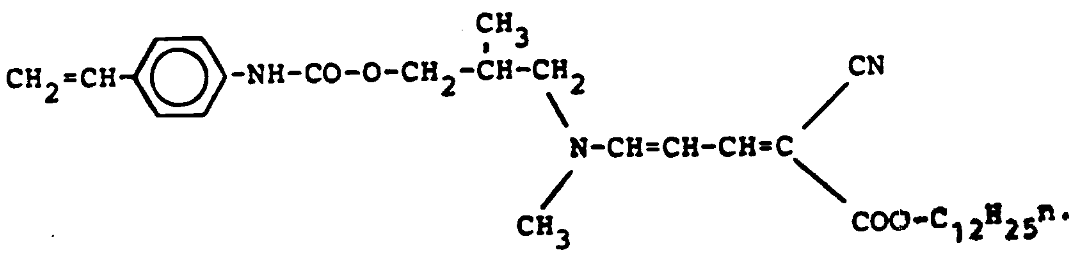

M 9 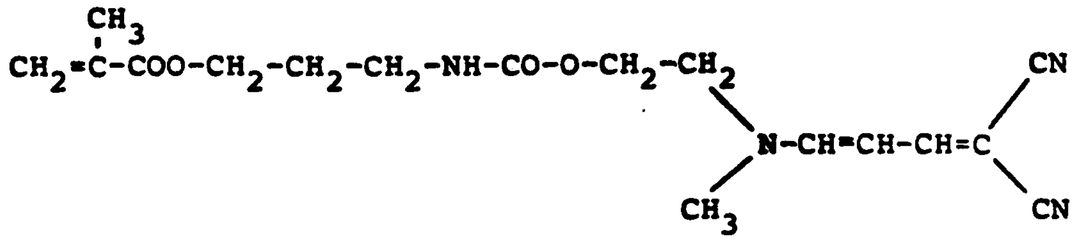

M 10 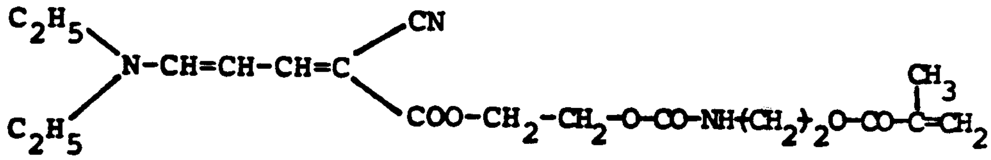

M 11 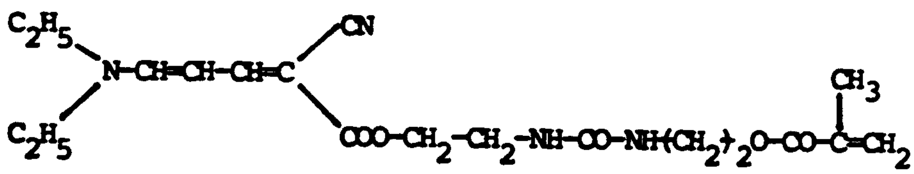

M 12 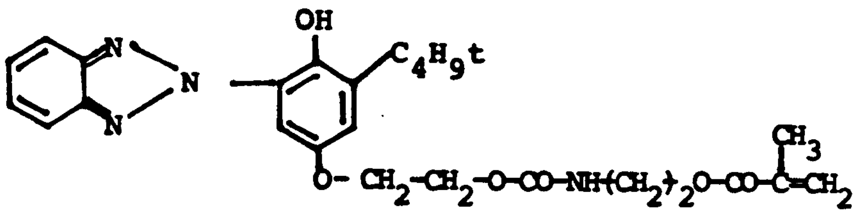

M 13 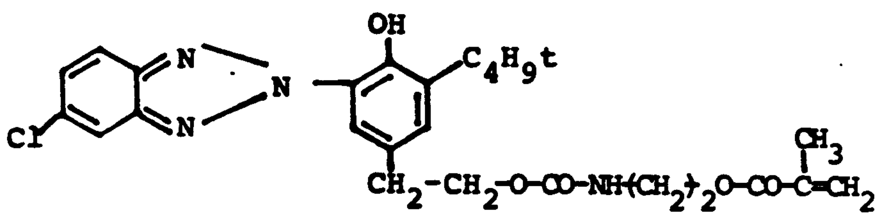

M 14 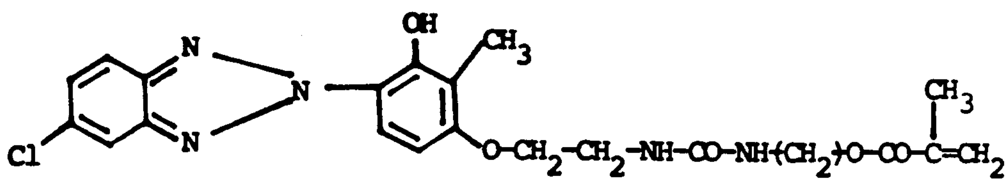

M 15 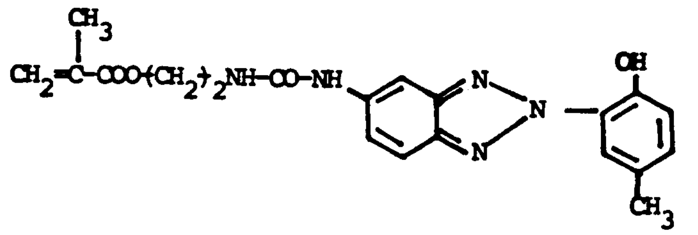

M 16 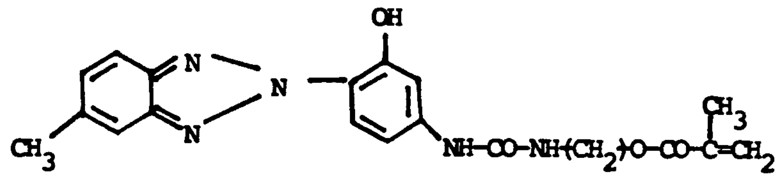

M 17 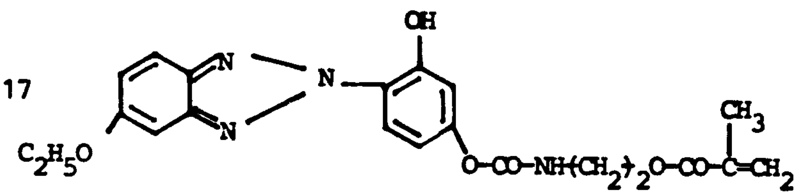

M 18 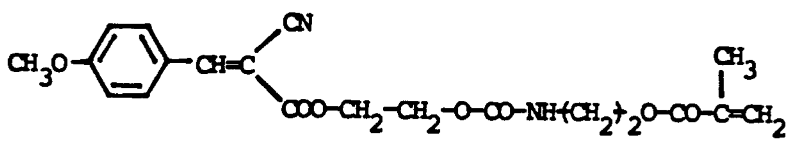

M 19 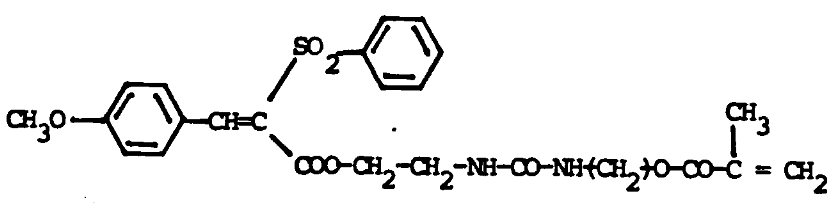

M 20 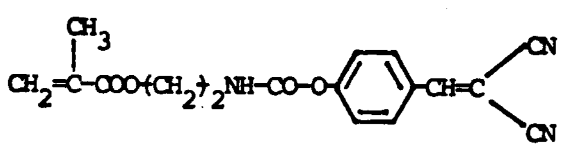

M 21 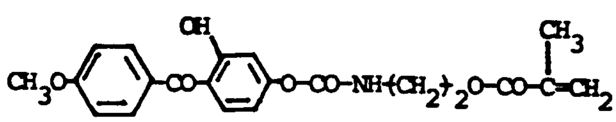

M 22 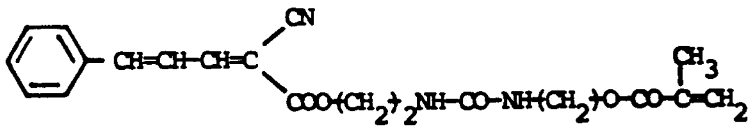

M 23 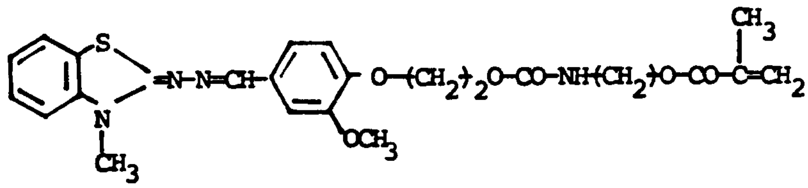

M 24 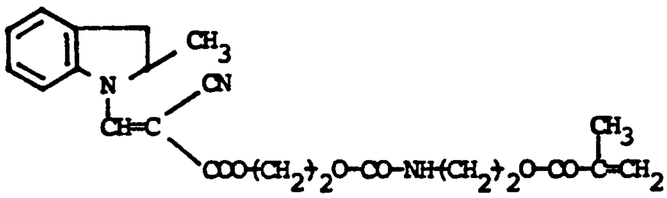

M 25 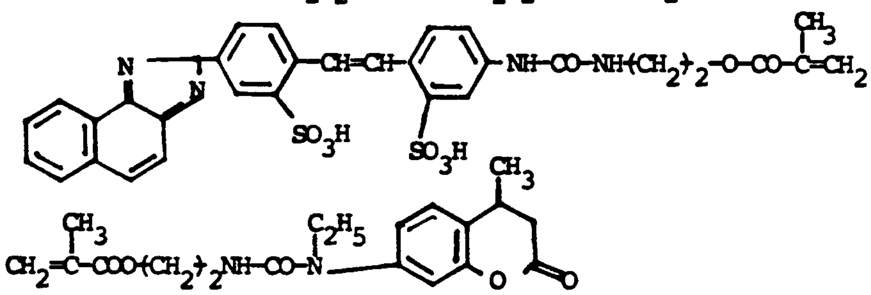

M 26

M 27 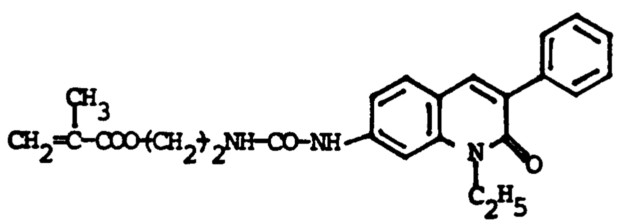

M 28 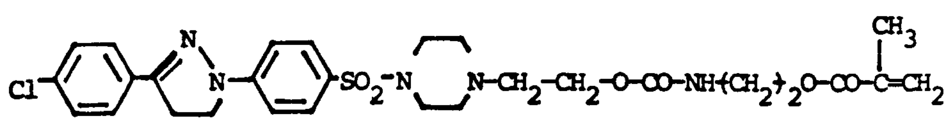

M 29

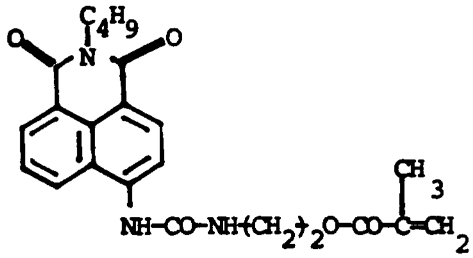

M 30

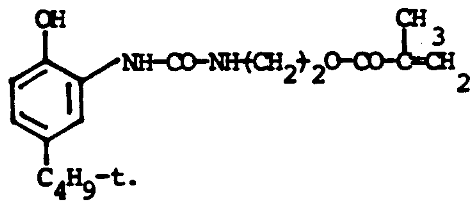

M 31

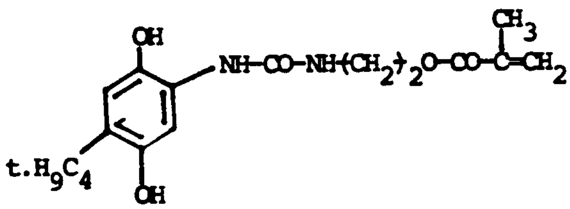

M 32

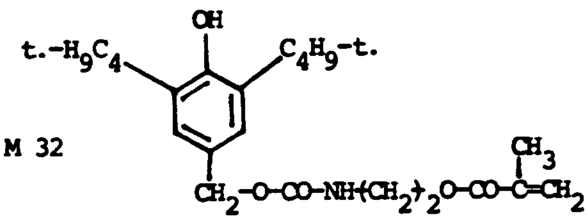

M 33

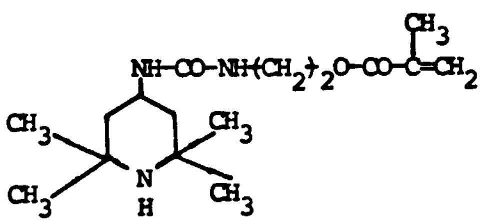

M 34

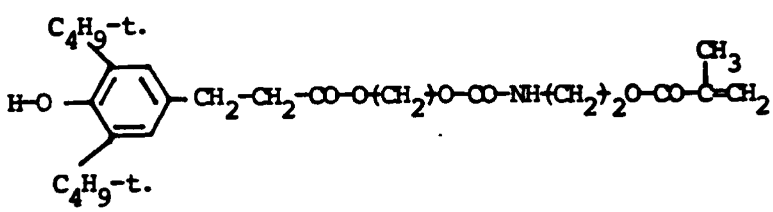

M 35

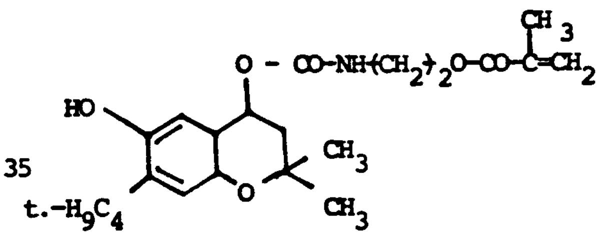

M 36

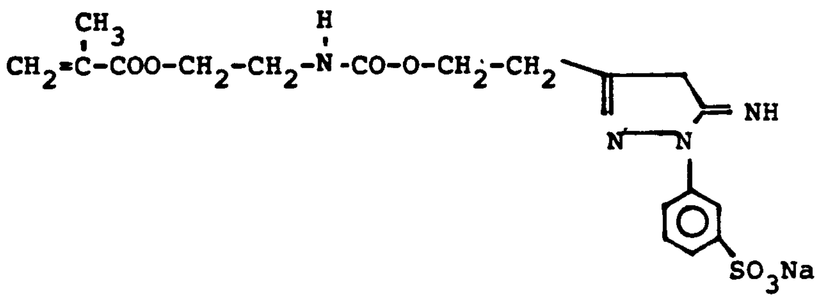

M 37

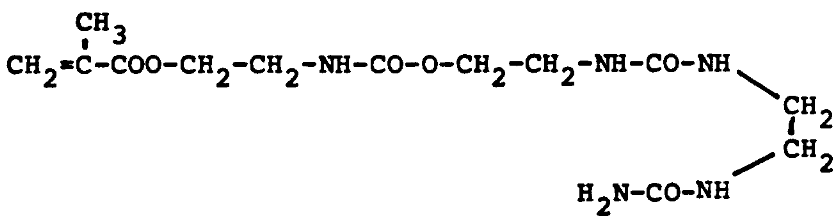

M 38

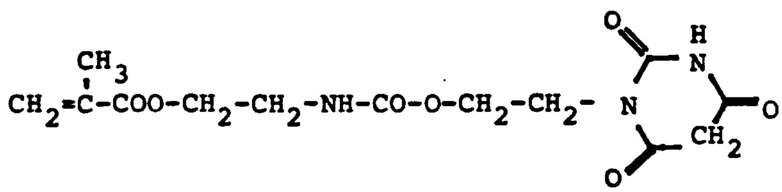

Zusätzlich zu den Ausgangsmonomeren M können weitere Monomere unter Erhalt von Copolymeren zur Polymerisation mitverwendet werden.

Beispiele für Monomere (Comonomere), die zur Copolymerisation mit dem Monomeren gemäß Formel Ia verwendet werden, umfassen einen Ester, vorzugsweise einen Niedrigalkylester und ein Amid, abgeleitet von einer Acrylsäure, beispielsweise Acrylsäure, einer $\alpha$-Chloracrylsäure, einer $\alpha$-Alkylacrylsäure wie Methacrylsäure, (beispielsweise Acrylamid, Methylmethacrylat, Ethylacrylat, Ethylmethacrylat, n-Propylacrylat, n-Butylacrylat, 2-Ethylhexylacrylat, n-Hexylacrylat, Octylmethacrylat, Laurylmethacrylat und Methylenbisacrylamid), ein Vinylester (beispielsweise Vinylacetat, Vinylpropionat und Vinyllaurat), Acrylnitril, Methacrylnitril, eine aromatische Vinylverbindung (beispielsweise Styrol und ein Derivat davon, wie Vinyltoluol, Divinylbenzol, Vinylacetophenon, Sulfostyrol und Styrolsulfonsäure), Itaconsäure, Zitraconsäure, Crotonsäure, Vinylidenchlorid, ein Vinylalkylether (beispielsweise Vinylethylether), ein Ester von Maleinsäure, N-Vinyl-2-pyrrolidon, N-Vinylpyridin und 2- und 4-Vinylpyridin.

Die Molekulargewichte der erfindungsgemäßen Verbindungen sind vorzugsweise größer als 5 000, insbesondere größer als 20 000, um eine ausreichende Diffusionsfestigkeit zu gewährleisten. Die obere Grenze ist unkritisch und kann, insbesondere wenn als Comonomer bi- oder polyfunktionelle Monomere verwendet werden, Werte von über 10 Millionen erreichen.

Es ist besonders bevorzugt, von diesen Monomeren einen Ester der Acrylsäure, einen Ester der Methacrylsäure und eine aromatische Vinylverbindung zu verwenden.

Zwei oder mehrere der vorstehend beschriebenen Comonomerverbindungen können miteinander verwendet werden. Beispielsweise ist es möglich, eine Kombination von n-Butylacrylat und Divinylbenzol, Styrol und Methylmethacrylat, Methylacrylat und Methacrylsäure zu verwenden.

Das ethylenisch ungesättigte Monomere, das zur Copolymerisation mit dem Monomeren gemäß Formel

Ia verwendet wird, kann ausgewählt werden derart, daß es sich günstig auf die physikalischen Eigenschaften und/oder chemischen Eigenschaften des herzustellenden Copolymeren auswirkt, beispielsweise die Löslichkeit, Verträglichkeit mit einem Bindemittel wie Gelatine in der fotografischen Kolloidzusammensetzung oder anderen fotografischen Zusätzen, beispielsweise bekannten fotografischen Ultraviolettstrahlen absorbierenden Mitteln, bekannten fotografischen Antioxidantien und bekannten Farbbild bildenden Mitteln, die Flexibilität und die thermische Stabilität davon.

In einer bevorzugten Ausführungsform werden die erfindungsgemäßen Polymeren als Polymerdispersion bzw. als Polymerlatex eingesetzt.

Der erfindungsgemäße Polymerlatex kann hergestellt werden nach einem Emulsionspolymerisationsverfahren oder durch Polymerisation eines Monomeren in einem organischen Lösungsmittel und anschließendes Dispergieren der Lösung in Latexform in einer wäßrigen Lösung von Gelatine.

Die frei-radikalische Polymerisation eines ethylenisch ungesättigten Monomeren wird initiiert durch Zusatz eines freien Radikals, das gebildet wird durch thermische Zersetzung eines chemischen Initiators, durch Einwirkung eines Reduktionsmittels auf eine oxidierende Verbindung (Redox-Initiator) oder durch physikalische Einwirkung, wie Bestrahlung mit Ultraviolettstrahlen oder anderen hochenergetischen Strahlungen, hohen Frequenzen

Beispiele für prinzipielle chemische Initiatoren umfassen ein Persulfat (beispielsweise Ammoniumpersulfat oder Kaliumpersulfat), Wasserstoffperoxid, ein Peroxid (beispielsweise Benzoylperoxid oder tert. Butylperoctoat) und eine Azonitrilverbindung (beispielsweise 4,4'-Azobis-4-cyanovaleriansäure und Azobisisobutyronitril).

Beispiele für konventionelle Redox-Initiatoren umfassen die Systeme Wasserstoffperoxid-Eisen(II)-salz; Kaliumpersulfat-Natriummetabisulfit und Cer IV-Salz-Alkohol.

Beispiele für die Initiatoren und deren Funktionen werden beschrieben von F.A. Bovey, in Emulsion Polymerization, Interscience Publishers Inc., New York, 1955, Seiten 59 bis 93.

Als Emulgator, der bei der Emulsionspolymerisation verwendet werden kann, wird eine Verbindung mit oberflächenaktiver Wirkung verwendet. Bevorzugte Beispiele dafür umfassen Seife, ein Sulfonat, ein Sulfat, eine kationische Verbindung, eine amphotere Verbindung und ein Schutzkolloid mit hohem Molekulargewicht. Spezielle Beispiele für die Emulgatoren und deren Funktionen werden beschrieben in Belgische Chemische Industrie, Bd. 28, Seiten 16 bis 20, 1963.

Ein besonderer Vorteil der erfindungsgemäß zu verwendenden Verbindungen besteht darin, daß sie bei der Emulsionspolymerisation nur geringe Emulgatormengen benötigen. In einer bevorzugten Ausführungsform wird, bezogen auf das Polymer, maximal 8 %, insbesondere maximal nur 6 % an Emulgator verwendet.

In einer anderen Anwendungsform wird das Polymere dispergiert. Dabei kann beim Dispergieren des Polymeren der Formel (I) in einer wäßrigen Gelatinelösung in Latexform ein organisches Lösungsmittel, das zum Auflösen verwendet wird, aus dem Gemisch vor der Überzugsbildung der Dispersion entfernt werden.

Als Lösungsmittel kommen solche in Frage, die ein gewisses Ausmaß an Wasserlöslichkeit aufweisen, so daß sie geeignet sind zur Entfernung durch Wäsche mit Wasser in einem Gelatinenudelzustand und solche, die durch Sprühtrocknen, Vakuum- oder Dampfspülen, entfernt werden können.

Außerdem umfassen Beispiele für die organischen Lösungsmittel, die zur Entfernung geeignet sind, einen Ester (beispielsweise einen Niedrigalkylester), einen Niedrigalkylether, ein Keton, einen halogenierten Kohlenwasserstoff (beispielsweise Methylenchlorid, Trichlorethylen), einen fluorierten Kohlenwasserstoff, einen Alkohol (beispielsweise Methylalkohol bis Butylalkohol) und eine Kombination davon.

Es kann jeglicher Typ von Dispergiermittel bei der Dispergierung erfindungsgemäßen Verbindungen verwendet werden. Ionische oberflächenaktive Mittel und insbesondere anionische oberflächenaktive Mittel sind jedoch bevorzugt.

Darüber hinaus ist es möglich, ampholytische oberflächenaktive Mittel zu verwenden, wie C-Cetylbetain, ein N-Alkylaminopropionat und ein N-Alkyliminodipropionat, usw.

Um die Dispersionsstabilität zu verbessern und die Flexibilität der aufgeschichteten Emulsion zu verbessern, kann eine geringe Menge (nicht mehr als 50 Gew.-% der wiederkehrenden Einheiten der Formel (I)) eines permanenten Lösungsmittels, nämlich eines mit Wasser nicht mischbaren organischen Lösungsmittels mit hohem Siedepunkt (d.h. über 200 ° C) beispielsweise Dibutylphthalat und/oder Trikresylphosphat zugesetzt werden. Es ist nötig, daß die Konzentration des permanenten Lösungsmittels ausreichend gering ist, um das Polymere zu plastifizieren, während es in einem Zustand eines festen Teilchens gehalten wird. Darüber hinaus ist es bei Verwendung eines permanenten Lösungsmittels bevorzugt, wenn dessen Menge so gering wie möglich ist, so daß die Dicke der endgültigen Emulsionsschicht oder der hydrophilen Kolloidschicht verringert wird, um eine gute Schärfe aufrechtzuerhalten.

Die erfindungsgemäß zu verwendenden Verbindungen können - in Abhängigkeit von der Natur des

Restes St - z.B. in einer Bindemittelschicht oder in einer lichtempfindlichen Silberhalogenidemulsionsschicht eingebracht werden. Handelt es sich bei St um eine gegen UV-Licht und sichtbares Licht schützende Struktur, wird die Verbindung vorzugsweise in einer oben liegenden Schicht eingebracht, z.B. in eine oberhalb aller lichtempfindlichen Schichten angeordneten Schicht.

Handelt es sich bei St um eine den Schleier drückende Struktur, wird die Verbindung vorzugsweise in eine Silberhalogenidemulsionsschicht eingebracht.

Die zu verwendende Menge hängt von dem Verwendungszweck ab und kann in üblicher Weise leicht optimiert werden. Bevorzugte Mengen sind z. B.:

Bei Einsatz als Schutz gegen UV- und sichtbares Licht: 50 - 1000 mg/m$^2$.

Bei Einsatz als Formalinfänger: 100 - 2000 mg/m$^2$ und insbesondere 0,5 - 12 Mol pro Mol Purpur-4-Äquivalentkuppler.

Bei Einsatz als Stabilisator oder Antischleiermittel gegen den Silberhalogenidschleier: 10 - 300 mg pro 100 g Silber, berechnet als $AgNO_3$.

Außer den bereits genannten Schichten können weitere, nicht lichtempfindliche Hilfsschichten in dem erfindungsgemäßen farbfotografischen Aufzeichnungsmaterial vorhanden sein, z.B. Haftschichten, Lichthofschutzschichten oder Deckschichten, insbesondere Zwischenschichten zwischen den lichtempfindlichen Schichten, wodurch die Diffusion von Entwickleroxidationsprodukten aus einer Schicht in eine andere wirksam unterbunden werden soll.

Die lichtempfindlichen Silberhalogenidemulsionsschichten können in Teilschichten unterschiedlicher Empfindlichkeit aufgespalten werden.

Den lichtempfindlichen Silberhalogenidemulsionsschichten sind Farbkuppler zugeordnet, die mit Farbentwickleroxidationsprodukten unter Bildung eines nichtdiffundierenden Farbstoffes reagieren. Zweckmäßigerweise sind die Farbkuppler nichtdiffundierend in der lichtempfindlichen Schicht selbst oder in enger Nachbarschaft hierzu untergebracht.

So kann die rotempfindliche Schicht beispielsweise einen nicht-diffundierenden Farbkuppler zur Erzeugung des blaugrünen Teilfarbenbildes enthalten, in der Regel einen Kuppler vom Phenol- oder $\alpha$-Naphtholtyp. Die grünempfindliche Schicht kann beispielsweise mindestens einen nicht-diffundierenden Farbkuppler zur Erzeugung des purpurnen Teilfarbenbildes enthalten, wobei üblicherweise Farbkuppler vom Typ des 5-Pyrazolons verwendet werden. Die blauempfindliche Schicht kann beispielsweise mindestens einen nicht-diffundierenden Farbkuppler zur Erzeugung des gelben Teilfarbenbildes, in der Regel einen Farbkuppler mit einer offenkettigen Ketomethylengruppierung enthalten. Bei den Farbkupplern kann es sich z.B. um 6-, 4- und um 2-Äquivalentkupplern handeln. Geeignete Kuppler sind beispielsweise bekannt aus den Veröffentlichungen "Farbkuppler" von W. Pelz in "Mitteilungen aus den Forschungslaboratorien der Agfa, Leverkusen/ München", Band III, Seite 111 (1961), K. Venkataraman in "The Chemistry of Synthetic Dyes", Vol. 4, 341 bis 387, Academic Press (1971) und T.H. James, "The Theory of the Photographic Process", 4. Ed., S. 353-362, sowie aus der Zeitschrift Research Disclosure Nr. 17643 vom Dezember 1978, Abschnitt VII, veröffentlicht von Industrial Opportunities Ltd., Homewell Havant, Hampshire, PO9 1 EF in Großbritannien.

Das Aufzeichnungsmaterial kann weiterhin DIR-Verbindungen und Weißkuppler, die bei Reaktion mit Farbentwickleroxidationsprodukten keinen Farbstoff ergeben, enthalten. Die von den DIR-Verbindungen abspaltbaren Inhibitoren können unmittelbar oder über nicht hemmende Zwischenverbindungen abgespalten werden. Verwiesen wird auf GB 953 454, US 3 632 345, US 4 248 962 und GB 2 072 363.

Die verwendeten lichtempfindlichen Silberhalogenidemulsionen können als Halogenid Chlorid, Bromid und Iodid bzw. Mischungen davon enthalten. In einer bevorzugten Ausführungsform besteht der Halogenidanteil wenigstens einer Schicht zu 0 bis 12 Mol-% aus AgI, zu 0 bis 50 Mol-% aus AgCl und zu 50 bis 100 % aus AgBr, wobei sich die Summe dieser Anteile zu 100 % ergänzt.

In einer bevorzugten Ausführungsform handelt es sich um überwiegend kompakte Kristalle, die z.B. kubisch oder oktaedrisch sind oder Übergangsformen aufweisen. Sie lassen sich dadurch kennzeichnen, daß sie im wesentlichen eine Dicke von mehr als 0,2 $\mu$m aufweisen. Das durchschnittliche Verhältnis von Durchmesser zu Dicke ist bevorzugt kleiner als 8:1, wobei gilt, daß der Duchmesser eines Kornes definiert ist als der Durchmesser eines Kreises mit einem Kreisinhalt entsprechend der projizierten Fläche des Kornes.

In einer anderen bevorzugten Ausführungsform können alle oder einzelne Emulsionen aber auch im wesentlichen tafelförmige Silberhalogenidkristalle aufweisen, bei denen das Verhältnis von Durchmesser zu Dicke größer als 8:1 ist.

Die Emulsionen können chemisch sensibilisiert sein. Zur chemischen Sensibilisierung der Silberhalogenidkörner sind die üblichen Sensibilisierungsmittel geeignet. Besonders bevorzugt sind schwefelhaltige Verbindungen, beispielsweise Allylisothiocyanat, Allylthioharnstoff und Thiosulfate.

Geeignet als chemische Sensibilisatoren sind auch Edelmetalle bzw. Edelmetallverbindungen wie Gold, Platin, Palladium, Iridium, Ruthenium oder Rhodium. Diese Methode der chemischen Sensibilisierung ist in dem Artikel von R. Koslowsky, Z.Wiss.Phot. 46, 65-72 (1951), beschrieben. Es ist ferner möglich, die Emulsionen mit Polyalkylenoxid-Derivaten zu sensibilisieren. Verwiesen wird weiter auf die oben angegebene Research Disclosure Nr. 17 643, Abschnitt III.

Die Emulsionen können in an sich bekannter Weise optisch sensibilisiert werden, z.B. mit den üblichen Polymethinfarbstoffen, wie Neutrocyaninen, basischen oder sauren Carbocyaninen, Rhodacyaninen, Hemicyaninen, Styrylfarbstoffen, Oxonolen und ähnlichen. Derartige Sensibilisatoren sind von F.M. Hamer in "The Cyanine Dyes and related Compounds", (1964), beschrieben. Verwiesen sei diesbezüglich insbesondere auf Ullmanns Enzyklopädie der technischen Chemie, 4. Auflage. Band 18, Seiten 431 ff und auf die oben angegebene Research Disclosure Nr. 17643, Abschnitt IV.

Zusätzlich zu den erfindungsgemäß zu verwendenden Verbindungen können die üblichen Antischleiermittel und Stabilisatoren verwendet werden. Als Stabilisatoren sind besonders geeignet Azaindene, vorzugsweise Tetra- oder Pentaazaindene, insbesondere solche, die mit Hydroxyl- oder Aminogruppen substituiert sind. Derartige Verbindungen sind z.B. in dem Artikel von Birr, Z.Wiss.Phot. 47, 1952), S. 2-58, beschrieben. Weitere geeignete Stabilisatoren und Antischleiermittel sind in der oben angegebenen Research Disclosure Nr. 17643 in Abschnitt IV angegeben.

Die Bestandteile des fotografischen Materials können nach üblichen, bekannten Methoden eingearbeitet werden. Wenn es sich um wasser- oder alkalilösliche Verbindungen handelt, können sie in Form von wäßrigen Lösungen, gegebenenfalls unter Zusatz von mit Wasser mischbaren organischen Lösungsmitteln wie Ethanol, Aceton oder Dimethylformamid, zugesetzt werden. Wenn sie wasser- bzw. alkaliunlöslich sind, können sie in an sich bekannter Weise in dispergierter Form in die Aufzeichnungsmaterialien eingearbeitet werden. Zum Beispiel kann man eine Lösung dieser Verbindungen in einem niedrig siedenden organischen Lösungsmittel direkt mit der Silberhalogenidemulsion oder zunächst mit einer wäßrigen Gelatinelösung vermischen und darauf das organische Lösungsmittel entfernen. Die so erhaltene Dispersion der jeweiligen Verbindung kann anschließend mit der Silberhalogenidemulsion vermischt werden. Gegebenenfalls verwendet man zusätzlich noch sogenannte Ölformer, in der Regel höhersiedende organische Verbindungen, die die zu dispergierenden Verbindungen in Form Öliger Tröpfchen einschließen. Verwiesen wird in diesem Zusammenhang beispielsweise auf die US-Patentschriften 2 322 027, 2 533 514, 3 689 271, 3 764 336 und 3 765 897. Es ist auch möglich, z.B. Kuppler in Form beladener Latices einzubauen, s. DE-OS 2 541 274 und EP-A 14 921. Weiterhin können die Bestandteile auch als Polymere im Material festgelegt werden, siehe z.B. DE-OS 2 044 992, US 3 370 952 und US 4 080 211.

Für die erfindungsgemäßen Materialien können die üblichen Schichtträger verwendet werden, z.B. Träger aus Celluloseestern, z.B. Celluloseacetat und aus Polyestern. Geeignet sind ferner Papierträger, die gegebenenfalls beschichtet sein können z.B. mit Polyolefinen, insbesondere mit Polyethylen oder Polypropylen. Verwiesen wird diesbezüglich auf die oben angegebene Research Disclosure Nr. 17643 Abschnitt XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Verwiesen wird auf die in der oben angegebenen Research Disclosure 17643 in Abschnitt IX angegebenen Bindemittel.

Die Schichten des fotografischen Materials können in der üblichen Weise gehärtet sein, beispielsweise mit Härtern des Epoxidtyps, des heterocyclischen Ethylenimins und des Acryloyltyps. Weiterhin ist es auch möglich, die Schichten gemäß dem Verfahren der deutschen Offenlegungsschrift 2 218 009 zu härten, um farbfotografische Materialien zu erzielen, die für eine Hochtemperaturverarbeitung geeignet sind. Es ist ferner möglich, die fotografischen Schichten bzw. die farbfotografischen Mehrschichtenmaterialien mit Härtern der Diazin-, Triazin- oder 1,2-Dihydrochinolin-Reihe zu härten oder mit Härtern vom Vinylsulfon-Typ. Weitere geeignete Härtungsmittel sind aus den deutschen Offenlegungsschriften 2 439 551, 2 225 230, 2 317 672 und aus der oben angegebenen Research Disclosure 17643, Abschnitt XI bekannt.

Weitere geeignete Zusätze werden in der Research Disclosure 17643 und in "Product Licensing Index" von Dezember 1971, Seiten 107 - 110 angegeben.

Geeignete Farbentwicklersubstanzen für das erfindungsgemäße Material sind insbesondere solche vom p-Phenylendiamintyp, z.B. 4-Amino-N,N-diethyl-anilinhydrochlorid; 4-Amino-3-methyl-N-ethyl-N-$\beta$-(methansulfonamido)-ethylanilinsulfathydrat; 4-Amino-3-methyl-N-ethyl-N-$\beta$-hydroxyethylanilinsulfat; 4-Amino-N-ethyl-N-(2-methoxyethyl)-m-toluidin-di-p-toluolsulfonsäure und N-Ethyl-N-$\beta$-hydroxyethyl-p-phenylendiamin. Weitere brauchbare Farbentwickler sind beispielsweise beschrieben in J.Amer.Chem.Soc. 73, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung

können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren, insbesondere z.B. Ethylendiamintetraessigsäure, Nitrilotriessigsäure, Iminodiessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Beispiele

Die in den folgenden Beispielen angegebenen Verbindungen haben folgende Struktur:

UV-1 (Vergleich)

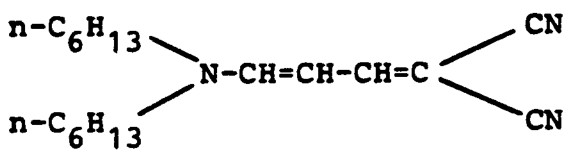

UV-2 (Vergleich)

Verbindung gemäß Example 1 der US-PS 4 307 184
(Acrylamid-diallylaminoallylidenmalonitril Copolymer)

UV-3 (Erfindung)

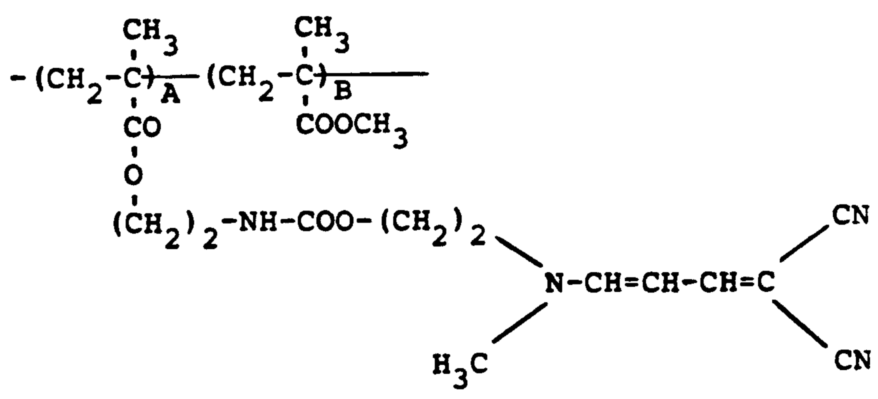

| | |
|---|---|
| Gewichtsanteil: | A 50 % |
| | B 50 % |

UV-4 (Erfindung)

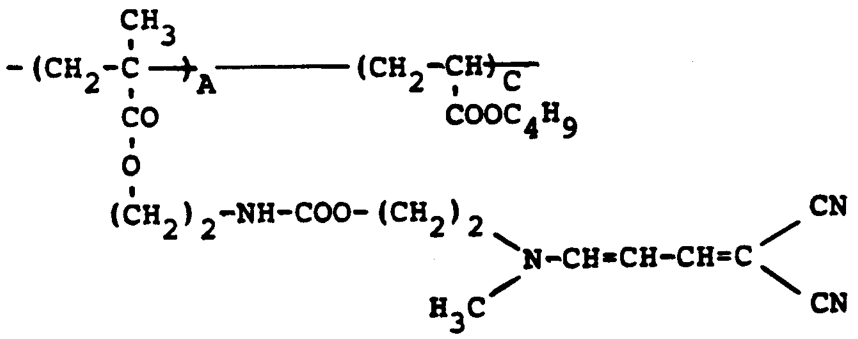

Gewichtsanteil: A = 50 %
C = 50 %

Die erfindungsgemäßen Verbindungen UV-3 und UV-4 wurden wie folgt hergestellt:

Polymerlatex UV-3

Unter Stickstoff werden 0,8 g einer 50 %igen Natriumdodecyldiphenyletherdisulfat-Lösung und 50 ml Wasser auf 70° C erwärmt. Dann gibt man 2,5 g einer Mischung aus 10 g Monomerverbindung M 1 und 10 g Methylmethacrylat. Dann tropft man eine Lösung aus 0,1 g Kaliumperoxodisulfat in 5 ml Wasser und 0,1 g Natriummetabisulfit in 5 ml Wasser zu und tropft nach 10 Minuten bei 70° C die restliche Menge des Monomergemisches zu. Nach 3 Stunden Rühren bei 70° C wird ein feinteiliger Latex mit einem Feststoffgehalt von 21,8 % erhalten.

Polymerlatex UV-4

Unter Stickstoff wurden 0,8 g einer 50 %igen Natriumdodecyldiphenyletherdisulfat-Lösung und 50 ml Wasser zusammen auf 70° C erwärmt. Dazu gibt man 2,5 g einer Mischung aus 10 g Monomerverbindung M 1 und 10 g Butylacrylat. Dann tropft man eine Lösung aus 0,1 g Kaliumperoxodisulfat in 5 ml Wasser und 0,1 g Natriummetabisulfit in 5 ml Wasser zu und nach 10 Minuten die restliche Menge des Monomergemisches. Nach 3 Stunden wird ein feinteiliger Latex mit einem Feststoffgehalt von 22,2 % erhalten.

Beispiel 1

Auf eine Triacetatunterlage wurde in üblicher Weise eine Gießlösung aufgetragen, die einen UV-Absorber, Gelatine und Na-Dodecylbenzolsulfonat enthielt. Es wurde soviel aufgetragen, daß jeweils 0,2 mMol des jeweiligen UV-Chromophors pro $m^2$ resultierte. Die Gelatinekonzentration wurde so eingestellt, daß pro $m^2$ 1 g Gelatine aufgetragen wurde. Auf diese UV-Absorberschicht wurde noch eine reine Gelatineschicht mit 1 g Gelatine pro $m^2$ aufgetragen. Von den UV-Absorbern wurden dabei zunächst Dispersionen durch Aufnahme der Verbindungen in Essigester und anschließendes Vermischen mit Gelatine mit Hilfe eines hochtourigen Rührwerkes und anschließendem Homogenisieren in einem Hochdruckhomogenisator hergestellt. Die hergestellten Folien wurden mit einer keinen UV-Absorber enthaltenden Folie als Referenz in einem Spektrometer vermessen. Es wurden die in Abbildung 1 wiedergegebenen UV-Spektren erhalten. Die in Abbildung 1 angegebenen Kurven bedeuten:

Kurve 1 Material mit UV-Absorber UV-1
Kurve 2 UV-Absorber UV-2
Kurve 3 UV-Absorber UV-3
Kurve 4 UV-Absorber UV-4

Es ist offensichtlich, daß die erfindungsgemäßen UV-Absorber UV-3 und UV-4 eine günstigere Absorption aufweisen.

Beispiel 2

Ein übliches farbfotografisches Negativmaterial wurde erhalten, indem auf einen üblichen Träger in der angegebenen Reihenfolge folgende Schichten aufgetragen wurden:
eine niedrigempfindliche rotempfindliche Schicht r

eine hochempfindliche rotempfindliche Schicht R
eine niedrigempfindliche grünempfindliche Schicht g
eine hochempfindliche grünempfindliche Schicht G
eine Gelbfilterschicht aus kolloidalem Silberfiltergelb
eine niedrigempfindliche blauempfindliche Schicht b
eine hochempfindliche blauempfindliche Schicht B
UV-Absorberschicht gemäß Beispiel 1
Gelatineschicht gemäß Beispiel 1

Die Proben wurden bildmäßig belichtet und einer üblichen Farbnegativentwicklung, wie sie beispielsweise aus der DE-A 3 029 209 bekannt ist, unterworfen. Es ergaben sich im Vergleich zu einer Probe, die keinen UV-Absorber enthielt, folgende Ergebnisse:

| Probe | UV-Absorber | Blau emfpindliche Schichten Empfindlichkeits-abnahme | $D_{min}$-Er-höhung | Braun wieder-gabe |
|---|---|---|---|---|
| 1 | UV-1 | 0,16 lg(Ixt) | 0,06 | Braun |
| 2 | UV-2 | 0,07 " | 0,01 | schmutzig violett |
| 3 | UV-3 | 0,11 " | 0,01 | Braun |
| 4 | UV-4 | 0,10 " | 0,01 | Braun |

Empfindlichkeitsabnahme:
Eine Vergrößerung der Angabe in 1g(Ixt) um 0,3 Einheiten entspricht einer Verdoppelung der Empfindlichkeit.

$D_{min}$: Minimale Farbdichte

Braunwiedergabe:
Es wurde eine braune Objektfarbe mit starker Remission im langwelligen UV aufgenommen. Die Wiedergabe wurde anhand einer neutralen Kopie beurteilt.

Aus der Tabelle folgt, daß mit den erfindungsgemäßen Proben 3 und 4 eine Verbesserung der Empfindlichkeit gegenüber Probe 1 und eine verbesserte Farbwiedergabe gegenüber Probe 2 erreicht wird.

**Patentansprüche**

**1.** Fotografisches Aufzeichnungsmaterial mit wenigstens einer lichtempfindlichen Silberhalogenidemulsionsschicht, gegebenenfalls weiteren Schichten sowie wenigstens einem in polymerer Form vorliegenden Stabilisator, dadurch gekennzeichnet, daß der Stabilisator wiederkehrende Struktureinheiten folgender Formel (I) aufweist

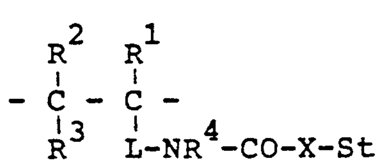

(I)

worin bedeuten

| | |
|---|---|
| $R^1$,$R^3$,$R^4$: | gleich oder verschieden, H oder Alkyl mit 1-4 C-Atomen |
| $R^2$ | : H oder Alkyl mit 1-4 C-Atomen oder $COOR^1$ |
| L | eine chemische Bindung oder ein zweiwertiges Bindeglied, |
| X | O oder $NR^4$ |
| St | einen Rest mit stabilisierender Wirkung. |

**2.** Aufzeichnungsmaterial nach Anspruch 1, dadurch gekennzeichnet, daß L steht für - $L^1$-$L^2$ - worin bedeuten

$L^1$ -$CONR^1$-,-$NR^1CO$-, -CO-, -COO-, -$SO_2$-, -O- oder eine chemische Bindung

$L^2$ gegebenenfalls substituiertes Alkylen mit 1 bis 8 C-Atomen, gegebenenfalls substituiertes Arylen oder eine chemische Bindung.

**3.** Material nach Anspruch 1, dadurch gekennzeichnet, daß die wiederkehrenden Struktureinheiten folgender Formel II entsprechen:

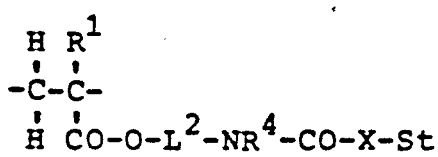

worin bedeuten:

$R^1$ H, $CH_3$

$R^4$ H, $CH_3$

$L^2$ Alkylen

St einen Rest mit stabilisierender Wirkung

X O oder $NR^1$.

**4.** Material nach Anspruch 1, dadurch gekennzeichnet, daß St eine UV-Licht absorbierende Einheit ist.

**5.** Material nach Anspruch 1, dadurch gekennzeichnet, daß der in polymerer Form vorliegende Stabilisator ein Copolymer ist.

**6.** Material nach Anspruch 1, dadurch gekennzeichnet, daß St eine UV-absorbierende Struktureinheit mit einer Allylidenmalonsäurenitrilgruppierung ist.

**7.** Material nach Anspruch 1, dadurch gekennzeichnet, daß die wiederkehrenden Struktureinheiten folgender Formel III entsprechen.

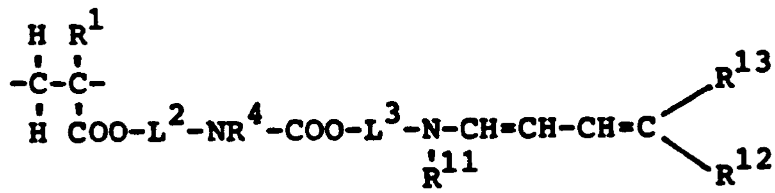

worin bedeuten:

$R^1$ H, $CH_3$,

$R^4$ H, $CH_3$,

$L^2$ und $L^3$ gleich oder verschieden, Ethylen, Propylen oder Butylen,

$R^{11}$ eine gegebenenfalls substituierte Alkylgruppe,

$R^{12}$ eine Cyanogruppe oder -$SO_2R^{14}$,

$R^{13}$ CN oder $COOR^{15}$,

$R^{14}/R^{15}$ gleich oder verschieden, eine gegebenenfalls substituierte Alkylgruppe mit 1 bis 20 C-Atomen oder eine gegebenenfalls substituierte Arylgruppe mit 6 bis 20 C-Atomen.

**8.** Material nach Anspruch 3, dadurch gekennzeichnet, daß der UV-Stabilisator über den lichtempfindlichen Silberhalogenidemulsionsschichten angeordnet ist.

**9.** Verbindungen mit wiederkehrenden Struktureinheiten folgender Formel (I)

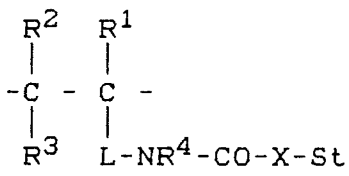

worin bedeuten

| | |
|---|---|
| $R^1$, $R^3$, $R^4$: | gleich oder verschieden, H oder Alkyl mit 1-4 C-Atomen |
| $R^2$: | H oder Alkyl mit 1-4 C-Atomen oder $COOR^1$ |
| L | eine chemische Bindung oder ein zweiwertiges Bindeglied, |
| X | O oder $NR^4$ |
| St | einen Rest mit stabilisierender Wirkung. |

## Claims

**1.** A photographic recording material comprising at least one photosensitive silver halide emulsion layer, optionally other layers and at least one stabilizer present in polymeric form, characterized in that the stabilizer contains recurring structural units corresponding to formula (I)

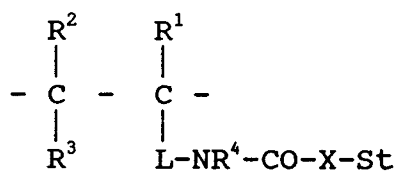

(I)

in which

| | |
|---|---|
| $R^1$, $R^3$, $R^4$ | may be the same or different and represent H or $C_{1-4}$ alkyl, |
| $R^2$ | represents H or $C_{1-4}$ alkyl or $COOR^1$, |
| L | is a chemical bond or a difunctional binding link, |
| X | is O or $NR^4$, |
| St | is a stabilizing function. |

**2.** A recording material as claimed in claim 1, characterized in that L stands for $-L^1-L^2-$ in which

| | |
|---|---|
| $L^1$ | represents $-CONR^1-$, $-NR^1CO-$, -CO-, -COO-, $-SO_2-$, -O- or a chemical bond, |
| $L^2$ | is optionally substituted $C_{1-8}$ alkylene, optionally substituted arylene or a chemical bond. |

**3.** A material as claimed in claim 1, characterized in that the recurring structural units correspond to formula II:

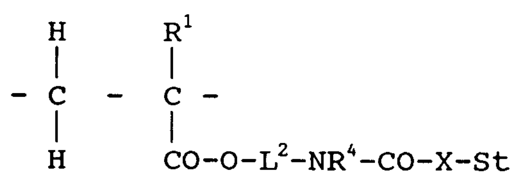

in which

| | |
|---|---|
| $R^1$ | represents H, $CH_3$, |
| $R^4$ | represents H, $CH_3$, |
| $L^2$ | represents alkylene. |
| St | is a stabilizing function, |
| X | represents O or $NR^1$. |

**4.** A material as claimed in claim 1, characterized in that St is a UV-absorbing unit.

**5.** A material as claimed in claim 1, characterized in that the stabilizer present in polymeric form is a copolymer.

**6.** A material as claimed in claim 1, characterized in that St is a UV-absorbing structural unit containing an allylidene malonic acid nitrile group.

**7.** A material as claimed in claim 1, characterized in that the recurring structural units correspond to formula III:

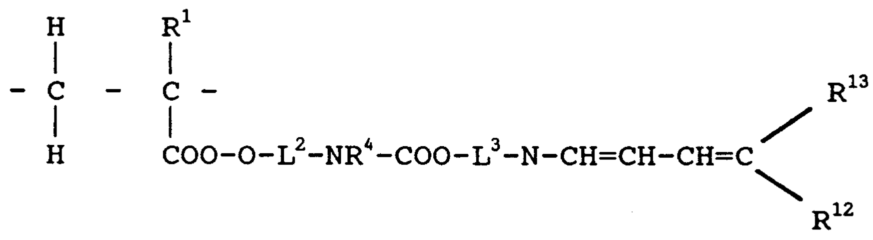

in which

| | |
|---|---|
| $R^1$ | represents H, $CH_3$, |
| $R^4$ | represents H, $CH_3$, |
| $L^2$ and $L^3$ | may be the same or different and represent ethylene, propylene or butylene, |
| $R^{11}$ | is an optionally substituted alkyl group, |
| $R^{12}$ | is a cyano group or $-SO_2R^{14}$, |
| $R^{13}$ | is CN or $COOR^{15}$, |
| $R^{14}/R^{15}$ | may be the same or different and represent an optionally substituted $C_{1-20}$ alkyl group or an optionally substituted $C_{6-20}$ aryl group. |

**8.** A material as claimed in claim 3, characterized in that the UV stabilizer is arranged above the photosensitive silver halide emulsion layers.

**9.** Compounds containing recurring structural units corresponding to formula (I)

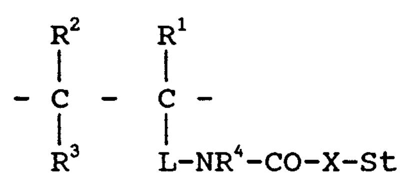

(I)

in which

| | |
|---|---|
| $R^1$, $R^3$, $R^4$ | may be the same or different and represent H or $C_{1-4}$ alkyl, |
| $R^2$ | represents H or $C_{1-4}$ alkyl or $COOR^1$, |
| L | is a chemical bond or a difunctional binding link, |
| X | is O or $NR^4$, |
| St | is a stabilizing function. |

**Revendications**

**1.** Matériau d'enregistrement photographique ayant au moins une couche d'émulsion d'halogénure d'argent sensible à la lumière, éventuellement d'autres couches ainsi qu'au moins un stabilisant présent sous forme polymère, caractérisé en ce que le stabilisant présente des motifs de structure récurrents de formule I suivante :

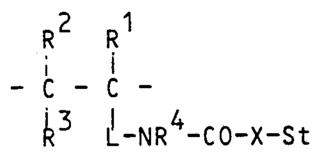

(I)

dans laquelle

| | |
|---|---|
| $R^1$, $R^3$, $R^4$ | sont identiques ou différents et représentent H ou un groupe alkyle en $C_1$-$C_4$ ; |
| $R^2$ | représente H ou un groupe alkyle en $C_1$-$C_4$ ou $COOR^1$ ; |
| L | représente une liaison chimique ou un chaînon divalent de liaison ; |
| X | représente O ou $NR^4$ ; |
| St | représente un reste ayant un effet stabilisant. |

**2.** Matériau d'enregistrement selon la revendication 1, caractérisé en ce que L représente $-L^1-L^2-$ dans lequel :

| | |
|---|---|
| $L^1$ | représente $-CONR^1-$, $-NR^1CO-$, -CO-, -COO-, $-SO_2-$, -O- ou une liaison chimique ; |
| $L^2$ | représente un groupe alkylène en $C_1$-$C_8$ éventuellement substitué, un groupe arylène éventuellement substitué ou une liaison chimique. |

**3.** Matériau selon la revendication 1, caractérisé en ce que les motifs de structure récurrents correspondent à la formule II suivante :

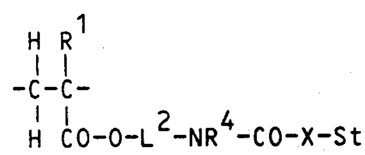

dans laquelle

| | |
|---|---|
| $R^1$ | représente H ou $CH_3$, |
| $R^4$ | représente H ou $CH_3$, |
| $L^2$ | représente un groupe alkylène, |
| St | représente un reste à effet stabilisant et |
| X | représente O ou $NR^1$. |

**4.** Matériau selon la revendication 1, caractérisé en ce que St est un motif absorbant la lumière UV.

**5.** Matériau selon la revendication 1, caractérisé en ce que le stabilisant présent sous forme polymère est un copolymère.

**6.** Matériau selon la revendication 1, caractérisé en ce que St est un motif de structure absorbant les UV ayant un groupe allylidènemalonitrile.

**7.** Matériau selon la revendication 1, caractérisé en ce que les motifs de structure récurrents correspondent à la formule III :

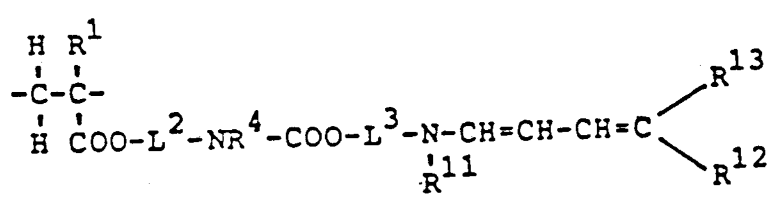

dans laquelle

| | |
|---|---|
| $R^1$ | représente H ou $CH_3$, |
| $R^4$ | représente H ou $CH_3$, |
| $L^2$ et $L^3$ | sont identiques ou différents et représentent un groupe éthylène, propylène ou butylène, |

$R^{11}$ représente un groupe alkyle éventuellement substitué,
$R^{12}$ représente un groupe cyano ou $-SO_2R^{14}$,
$R^{13}$ représente un groupe CN ou $COOR^{15}$,
$R^{14}/R^{15}$ sont identiques ou différents et représentent chacun un groupe alkyle en $C_1$-$C_{20}$ éventuellement substitué ou un groupe aryle en $C_6$-$C_{20}$ éventuellement substitué.

**8.** Matériau selon la revendication 3, caractérisé en ce que le stabilisant anti-UV est disposé sur les couches d'émulsion d'halogénure d'argent sensibles à la lumière.

**9.** Composés à motifs de structure récurents de formule I suivante :

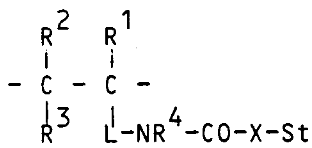

dans laquelle
$R^1$, $R^3$, $R^4$ sont identiques ou différents et représentent H ou un groupe alkyle en $C_1$-$C_4$ ;
$R^2$ représente H ou un groupe alkyle en $C_1$-$C_4$ ou $COOR^1$ ;
L représente une liaison chimique ou un chaînon divalent de liaison ;
X représente O ou $NR^4$ ;
St représente un reste ayant un effet stabilisant.

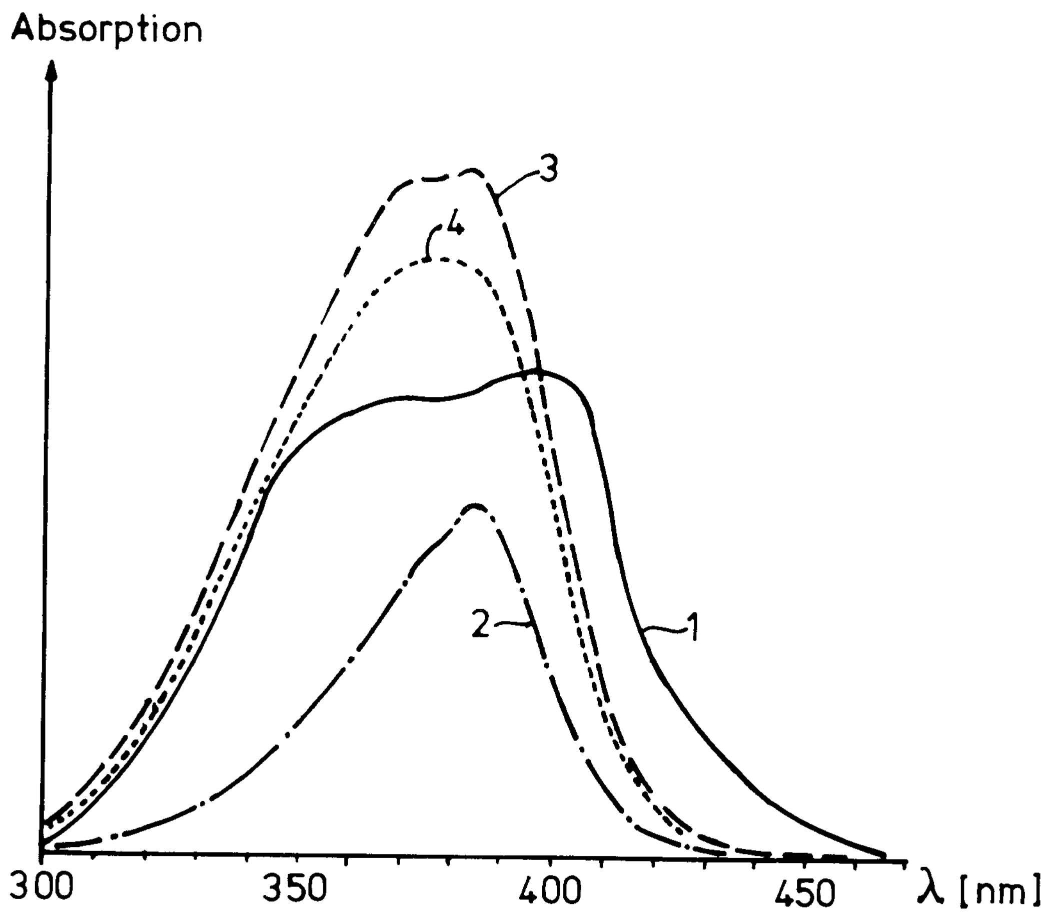
Absorption
3
4
1
2
300
350
400
450
λ [nm]